# EUROPEAN PATENT SPECIFICATION

(11) **EP 1 457 221 B1**
(45) Date of publication and mention of the grant of the patent: **08.08.2007**
(21) Application number: 03251560.3
(22) Date of filing: 14.03.2003
(51) Int. Cl.: A61M 5/32

(54) **Disposable syringe**
Einwegspritze
Seringue jetable

(43) Date of publication of application: 15.09.2004
(73) Proprietor: Shue, Ming-Jeng, Hsi Dist, Taichung City (TW); Shue, Phillip, Taichung City (TW)
(72) Inventor: Shue, Ming-Jeng, Hsi Dist, Taichung City (TW); Shue, Phillip, Taichung City (TW)
(74) Representative: Eddowes, Simon

(56) References cited:
- WO-A-00/20058
- WO-A-02/098480
- DE-U- 8 805 390
- GB-A- 2 270 471
- US-A- 4 840 185
- US-A- 5 445 620
- US-A- 5 531 706

## Description

This invention relates to a disposable syringe, more particularly to a disposable syringe which enables a needle cannula to be retracted within an encasing member.

Exposed needles of conventional disposable syringes may prick the fingers of health care workers after use, and may infect them with contagious fatal diseases, such as AIDS, hepatitis, etc. To reduce the risk of accidents, a disposable syringe with a retractable needle has been developed. However, such a disposable syringe suffers from the following disadvantages in actual use:
1. After injection, a plunger of the disposable syringe has to be first pushed forwardly to couple with the needle and then pulled rearwardly so as to pull the needle into the syringe body. Thereafter, the plunger has to be broken at a certain point to prevent inadvertent pressing of the plunger, which may cause the needle to project from the syringe body once again. Such a retracting procedure is relatively complicated, and considerations have to be given to whether the plunger can be firmly coupled to the needle in order to pull the needle that engages the front end of the syringe body. The coupling of the plunger to the needle and the subsequent pulling of the needle through the plunger are difficult to conduct since the engagement between the needle and the syringe body poses a certain resistance.
2. In order to facilitate retraction of the needle, a greater number of component parts are provided to couple the plunger to the needle, which, however, increases costs and complicates assembly.
3. Due to the provision of more component parts, the amount of residual medication of blood trapped in dead corners of the disposable syringe also increases to result in aggravated environmental pollution problem.

US 484 0185, US 553 1706, US 544 5620 and WO 020 98 480 describe syringes of various types, none of which disclose an arrangement where the female screw thread segment of the retaining member is a spirally extending groove, which advantageously prevents undesirable excessive angular displacement of the protrusions, thus making it easier to retract the needle after use.

The object of the present invention is to provide a disposable syringe which as a relatively simple construction and which enables retraction of a needle after use in a simple operating manner.

According to this invention, the disposable syringe includes a syringe body, a plunger, an encasing member and a retaining member.

The syringe body includes a surrounding barrel (23) which has an axis, front and rear surrounding ends opposite to each other in a longitudinal direction parallel to the axis, an outer surrounding wall surface, and an inner surrounding wall surface opposite to the outer surrounding wall surface. The inner surrounding wall surface confines an accommodation chamber. A front end wall extends from the front surrounding end radially and towards the axis to confine an internal port that is in fluid communication with the accommodation chamber. A needle cannula is disposed to extend in the longitudinal direction. A needle hub is disposed to secure the needle cannula to the front end wall such that the needle cannula is in fluid communication with the accommodation chamber through the internal port in the longitudinal direction.

The plunger includes a head portion which is disposed to be movable in the accommodation chamber and which is in slidable contact with the inner surrounding wall surface, and a stem portion which extends from the head portion in the longitudinal direction and outwardly of the rear surrounding end so as to be manually operated.

The encasing member is configured to be sleevable on the syringe body from the needle hub, and includes surrounding front and rear segments. The surrounding front segment surrounds the needle hub, and permits the needle cannula to extend outwardly thereof and to be retractable relative thereto. The surrounding rear segment extends from the surrounding front segment in the longitudinal direction, is to be sleeved on the surrounding barrel wall, and has an inner tubular wall surface which confronts and which is movable relative to the outer surrounding wall surface between a position of use, where the front end wall is closer to the surrounding front segment, and where the needle cannula extends outwardly of the surrounding front segment, and a disposal position, where the front end wall is remote from the surrounding front segment, and where the needle cannula is disposed inwardly of the surrounding front segment as a result of withdrawal of the needle hub from the surrounding front segment.

The retaining member is disposed between the inner tubular wall surface and the outer surrounding wall surface to arrest unforced movement of the surrounding barrel wall in the position of use.

Other features and advantages of the present invention will become apparent in the following detailed description of the preferred embodiments of the invention, with reference to the accompanying drawings, in which:
Fig. 1 is an exploded sectional view of the first preferred embodiment of a disposable syringe according to this invention;
Fig. 2 is a schematic view of the first preferred embodiment;
Fig. 3 is a sectional view of the first preferred embodiment in a state of use;
Fig. 4 is a sectional view of the first preferred embodiment in a disposal state;
Fig. 5 is a schematic view of the second preferred embodiment of a disposable syringe according to this invention;
Fig. 6 is a schematic view of the second preferred embodiment in a disposal state;
Fig. 7 is a schematic view of the third preferred embodiment of a disposable syringe according to this invention;
Fig. 8 is a sectional view of the fourth preferred embodiment of a disposable syringe according to this invention; and
Fig. 9 is a sectional view of the fourth preferred embodiment in a disposal state.

Before the present invention is described in greater detail, it should be noted that same reference numerals have been used to denote like elements throughout the specification.

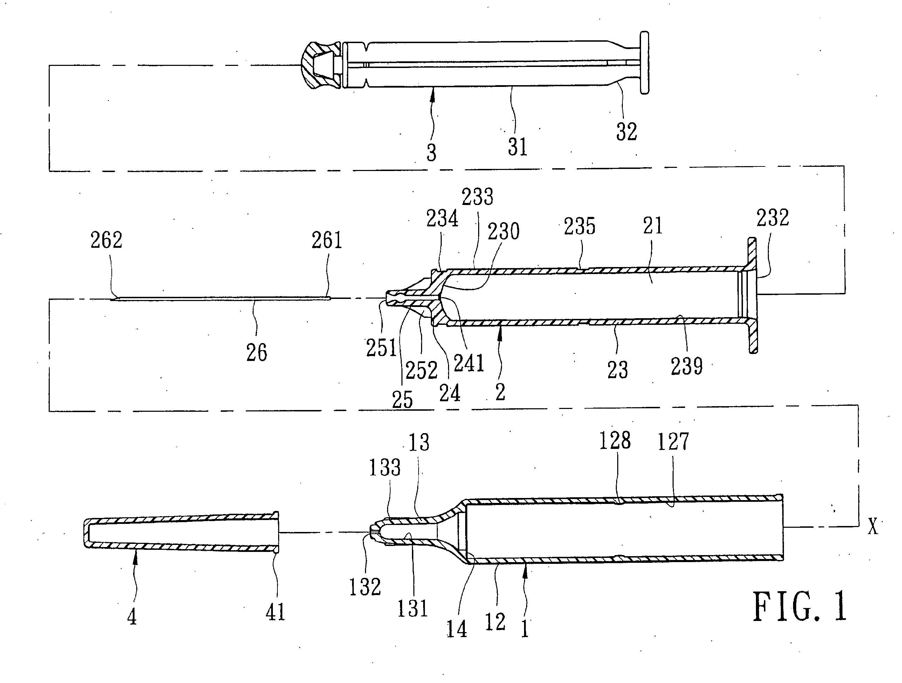

Referring to Figs. 1 to 3, the first preferred embodiment of a disposable syringe according to the present invention is shown to comprise a syringe body 2, a plunger 3, an encasing member 1, a retaining member, and a tip protector 4.

The syringe body 2 includes a surrounding barrel wall 23, a front end wall 24, a needle cannula 26, and a needle hub 25. The surrounding barrel wall 23 defines an axis (X), and has front and rear surrounding ends 230,232 opposite to each other in a longitudinal direction parallel to the axis (X) . The surrounding barrel wall 23 has an outer surrounding wall surface 233 and an inner surrounding wall surface 239 opposite to the outer surrounding wall surface 233 . The inner surrounding wall surface 239 confines an accommodation chamber 21. The front end wall 24 extends from the front surrounding end 230 radially and towards the axis (X) to confine an internal port 241 that is in fluid communication with the accommodation chamber 21. An annular retaining groove 234 is formed in the outer surrounding wall surface 233 adjacent to the front end wall 24.

The needle hub 25 is integrally formed with the front end wall 24, and extends from the front end wall 24 in the longitudinal direction to terminate at an insert end 251. The needle cannula 26 includes a secured end 261 which is secured to the needle hub 25 from the insert end 251, and a cannula body which extends from the secured end 261 in the longitudinal direction to terminate at a tip end 262 that is disposed outwardly of the needle hub 25 such that the needle cannula 26 is in fluid communication with the accommodation chamber 21 through the internal port 241. The needle hub 25 further has a plurality of ribs 252 which are displaced angularly from one another about the axis (X) , and each of which extends radially and outwardly.

The plunger 3 includes a head portion 31 which is disposed to be movable in the accommodation chamber 21 and which is in slidable contact with the inner surrounding wall surface 239, and a stem portion 32 which extends from the head portion 31 in the longitudinal direction and outwardly of the rear surrounding end 232 of the surrounding barrel wall 23 so as to be manually operated.

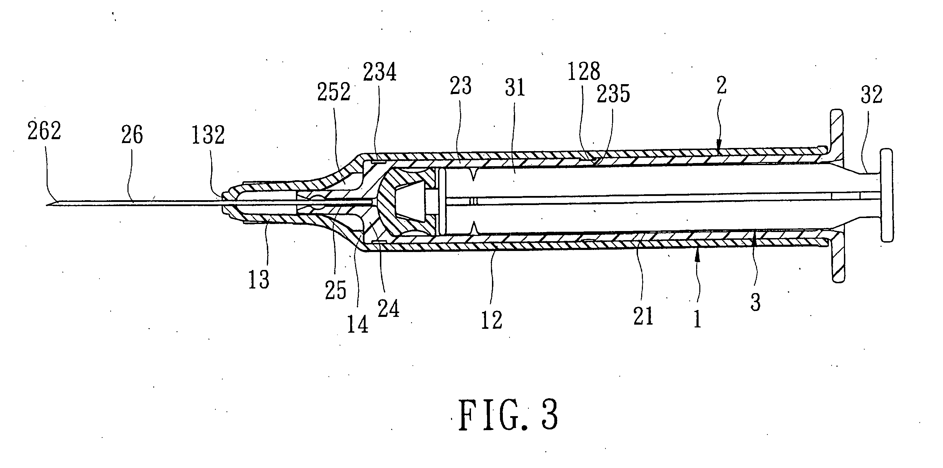

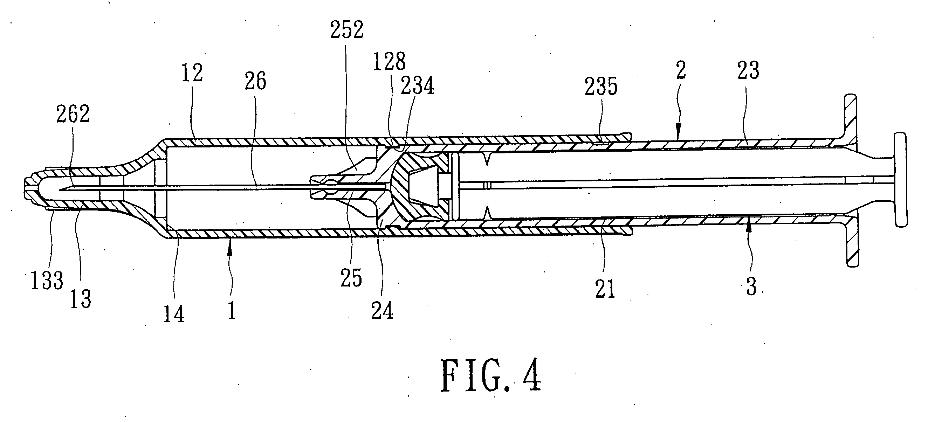

The encasing member 1 is sleevable on the syringe body 2 from the needle hub 25. The encasing member 1 includes a surrounding front segment 13 and a surrounding rear segment 12 which extends from the surrounding front segment 13 in the longitudinal direction. The surrounding front and rear segments 13,12 have a smaller-diameter inner front wall surface 131 and a larger-diameter inner tubular wall surface 127, respectively, so as to form a surrounding shoulder portion 14 therebetween. The surrounding front segment 13 surrounds the needle hub 25, and has a front open end 132 to permit the needle cannula 26 to extend outwardly thereof and to be retractable relative thereto. The surrounding rear segment 12 is sleeved on the surrounding barrel wall 23. Accordingly, the inner tubular wall surface 127 of the surrounding rear segment 12 confronts and is movable relative to the outer surrounding wall surface 233 of the surrounding barrel wall 23 between a position of use, as shown in Fig. 3, where the front end wall 24 abuts against the surrounding shoulder portion 14, and where the tip end 262 of the needle cannula 26 extends outwardly of the front open end 132 of the front surrounding segment 13, and a disposal position, as shown in Fig. 4, where the front end wall 24 is remote from the surrounding front segment 13, and where the tip end 262 of the needle cannula 26 is disposed inwardly of the surrounding front segment 13 as a result of withdrawal of the needle hub 25 from the surrounding front segment 13. Moreover, when the surrounding front segment 13 is brought to be sleeved on the needle hub 25, the ribs 252 of the needle hub 25 can create an increased friction force at the inner front wall surface 131, thereby establishing greater friction engagement between the needle hub 25 and the surrounding front segment 13.

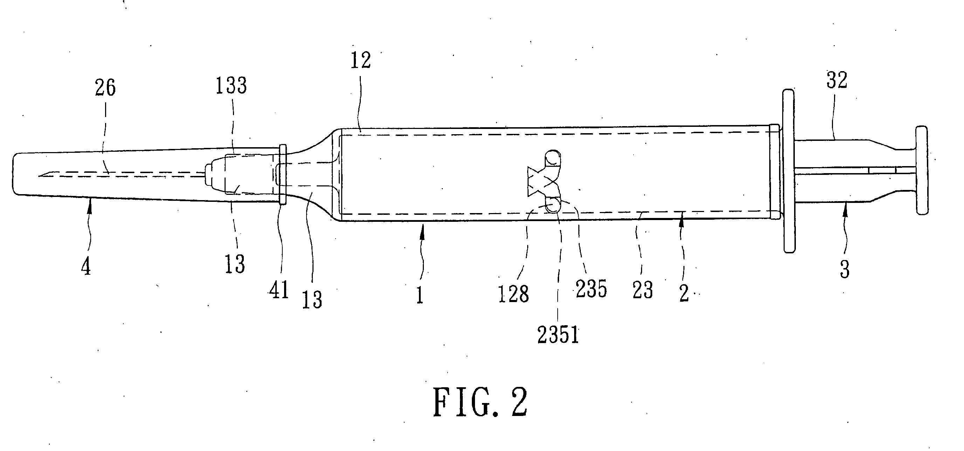

In addition, referring to Figs. 1 and 2, an outer front wall surface of the surrounding front segment 13 has a rib portion 133 which extends in the longitudinal direction. The tip protector 4 has a sleeve end 41 which is disposed to sleeve on the outer front wall surface of the surrounding front segment 13. The sleeve end 41 includes a groove portion which mates with the rib portion 133 to result in a splined engagement between the tip protector 4 and the outer front wall surface of the surrounding front segment 13, thereby ensuring secure shielding of the needle cannula 26.

The retaining member includes male and female screw thread segments 128,235 which are disposed on the inner tubular wall surface 127 of the surrounding rear segment 12 and the outer surrounding wall surface 233 of the surrounding barrel wall 23, respectively. The male screw thread segment 128 includes a plurality of angularly displaced ribs and surrounds the axis (X) . The female screw thread segment 235 is disposed opposite to the surrounding retaining groove 234 in the longitudinal direction, and includes a plurality of spirally extending grooves (as shown in Fig. 2).

When the encasing member 1 is brought over the needle hub 25 and is sleeved on the syringe body 2, the male screw thread segment 128 is angularly engageable with the female screw thread segment 235 by rotation of the syringe body 2 relative to the encasing member 1 in a counterclockwise direction, until the ribs of the male screw thread segment 128 reach ends 2351 of the grooves of the female screw thread segment 235 so as to restrain the surrounding barrel wall 23 from displacing along the surrounding rear segment 12 in the longitudinal direction, thereby arresting unforced movement of the surrounding barrel wall 23 in the position of use. Therefore, an injection procedure of medication fluid in the accommodation chamber 21 can be performed by pushing the stem portion 32 of the plunger 3 towards the front end wall 24.

When the injection procedure has been completed, referring to Figs. 2 to 4, the surrounding barrel wall 23 is angularly moved in a clockwise direction relative to the surrounding rear segment 12 so as to disengage the ribs of the male screw thread segment 128 from the grooves of the female screw thread segment 235, and is displaced in the longitudinal direction remote from the surrounding front segment 13. Subsequently, the surrounding barrel wall 23 is pulled in the same direction so as to bring the surrounding barrel wall 23 from the position of use to the disposal position, as shown in Fig. 4, such that the male screw thread segment 128 engages the surrounding retaining groove 234, thereby restraining the surrounding barrel wall 23 from displacing along the surrounding rear segment 12. In this position as described above, the used needle cannula 26 is disposed within the encasing member 1 for safe disposal.

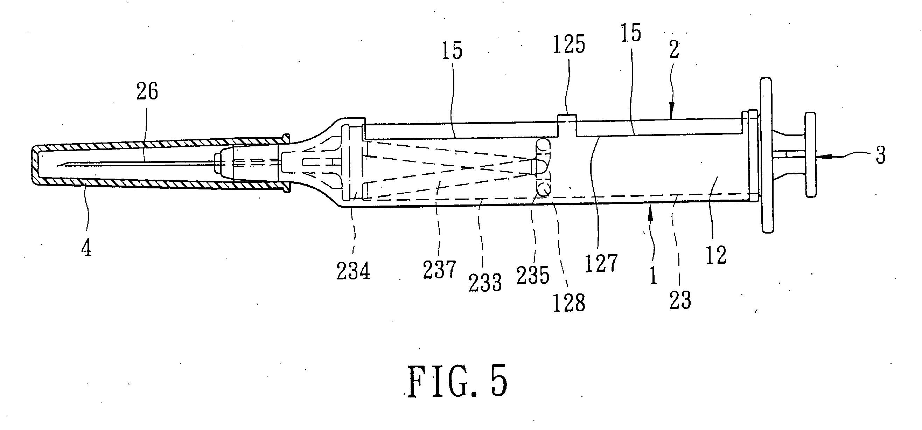

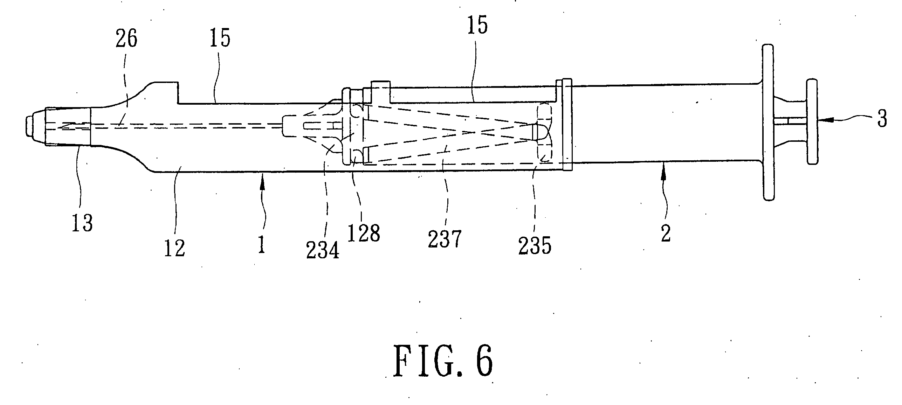

Referring to Figs. 5 and 6, the second preferred embodiment of the disposable syringe according to this invention is shown to be similar to the aforesaid embodiment in construction. The disposable syringe of this embodiment further includes a guideway 237 which is disposed on the outer surrounding wall surface 233 of the surrounding barrel wall 23, and which spirally extends to interconnect the female screw thread segment 235 and the surrounding retaining groove 234. As such, the male screw thread segment 128 is angularly movable along the guideway 237 between the female screw thread segment 235 and the surrounding retaining groove 234 so as to facilitate the displacement of the surrounding barrel wall 23 from the position of use to the disposal position.

In addition, the surrounding rear segment 12 of the encasing member 1 further has an outer tubular wall surface 125 radially opposite to the inner tubular wall surface 127, and a plurality of slots 15 which are disposed in the outer tubular wall surface 125, which are spaced apart from each other in the longitudinal direction, and which extend through the inner tubular wall surface 127 so as to expose a portion of the surrounding barrel wall 23 of the syringe body 2.

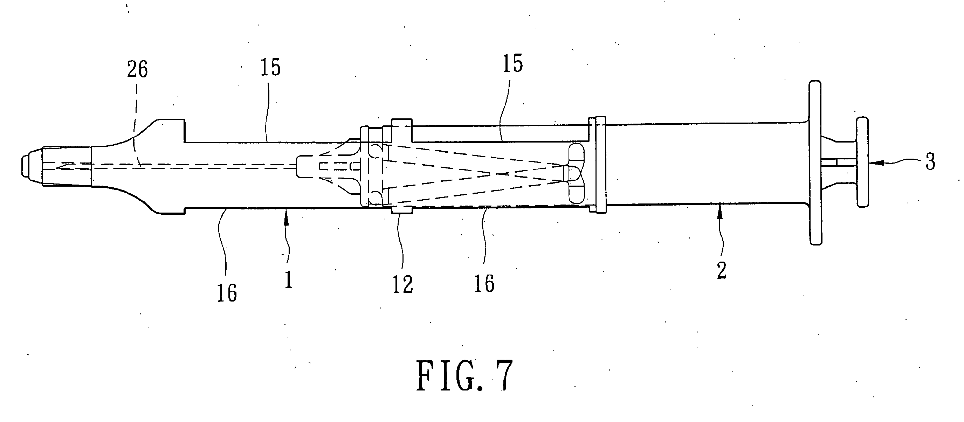

Referring to Fig. 7, the third preferred embodiment of the disposable syringe according to this invention is shown to be similar to the second embodiment in construction. In this embodiment, additional slots 16 are formed through the surrounding rear segment 12 of the encasing member 1, and are disposed diametrically opposite to the slots 15.

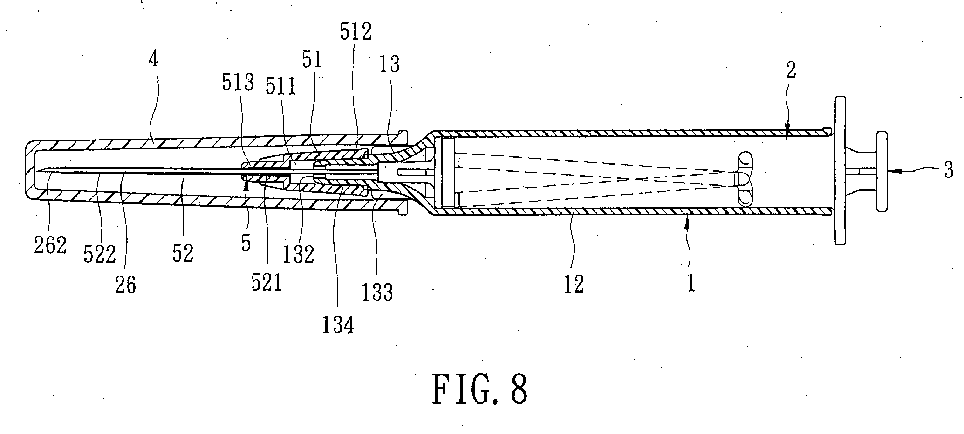

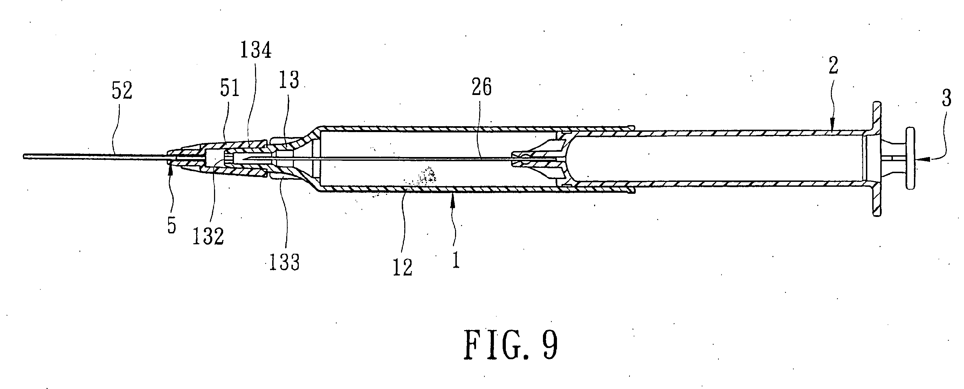

Referring to Figs. 8 and 9, the fourth preferred embodiment of the disposable syringe according to this invention is shown to be similar to the aforesaid embodiments in construction. The disposable syringe of this embodiment further comprises a catheter device 5 which includes a catheter hub 51 and a tubular catheter 52. In addition, the rib portion 133 of the surrounding front segment 13 is shorter than that of the aforesaid embodiment so that a front surrounding sleeved region 134 is formed on the surrounding front segment 13 and forwardly of the rib portion 133. The catheter hub 51 includes a surrounding hub wall which confines a duct 511 that extends along the axis, and which has a sleeve portion 512 that is sleeved on the front surrounding sleeved region 134, and a tip portion 513 opposite to the sleeve portion 512. The tubular catheter 52 includes a proximate segment 521 which is disposed in the tip portion 513 and which extends along the axis to communicate fluidly with the duct 511, and a distal segment 522 which extends from the proximate segment 521 along the axis to project outwardly of the tip portion 513. The cannula body of the needle cannula 26 passes through the tubular catheter 52 to permit the tip end 262 to project outwardly of the distal segment 522. Hence, after the encasing member 1, in which the syringe body 2 is in the disposal position, has been removed from the catheter hub 51, a transfusion member (not shown) with medication fluid or an empty barrel (not shown) can be connected to the catheter hub 51 for administering the medication fluid into a patient's vein or for drawing blood.

As illustrated, the disposable syringe of this invention has the following advantages:
1. By rotation of the syringe body 2 relative to the encasing member 1 in the clockwise direction to displace the surrounding barrel wall 23 from the position of use to the disposal position, the used needle cannula 26 can be retracted into the encasing member 1, thereby facilitating safe disposal of the disposable syringe.
2. The disposable syringe has a simple construction that is easy to fabricate and assemble at a relatively low cost.
3. Since the plunger 3 is in fit contact with the inner surrounding wall surface 239 of the surrounding barrel wall 23 during the injection procedure, and since there is no component part on the inner surrounding wall surface 239, the amount of residual medication or blood trapped in the accommodation chamber 21 can be minimized.
4. The disposable syringe can serve as an intravenous catheter inserting device for administering medication fluid into a patient's vein or for drawing blood.

## Claims

1. A disposable syringe comprising:
a syringe body (2) including
a surrounding barrel wall (23) which has an axis (X), front and rear surrounding ends (230,232) opposite to each other in a longitudinal direction parallel to the axis (X) , an outer surrounding wall surface (233), and an inner surrounding wall surface (239) opposite to said outer surrounding wall surface (233), said inner surrounding wall surface (239) confining an accommodation chamber (21),
a front end wall (24) which extends from said front surrounding end (230) radially and towards the axis (X) to confine an internal port (241) in fluid communication with said accommodation chamber (21),
a needle cannula (26) disposed to extend in the longitudinal direction, and
a needle hub (25) disposed to secure said needle cannula (26) to said front end wall (24) such that said needle cannula (26) is in fluid communication with said accommodation chamber (21) through said internal port (241) in the longitudinal direction; and
a plunger (3) including a head portion (31) disposed to be movable in said accommodation chamber (21) and in slidable contact with said inner surrounding wall surface (239) , and a stem portion (32) extending from said head portion (31) in the longitudinal direction and outwardly of said rear surrounding end (232) so as to be manually operated, said disposable syringe being **characterized by**:
an encasing member (1) which is configured to be sleevable on said syringe body (2) from said needle hub (25), and which includes a surrounding front segment (13) that surrounds said needle hub (25) , and that permits said needle cannula (26) to extend outwardly thereof and to be retractable relative thereto, and
a surrounding rear segment (12) extending from said surrounding front segment (13) in the longitudinal direction, to be sleeved on said surrounding barrel wall (23) , and having an inner tubular wall surface (127) , said inner tubular wall surface (127) confronting and being movable relative to said outer surrounding wall surface (233) between a position of use, where said front end wall (24) is closer to said surrounding front segment (13), and where said needle cannula 26) extends outwardly of said surrounding front segment (13), and a disposal position, where said front end wall (24) is remote from said surrounding front segment (13), and where said needle cannula 26) is disposed inwardly of said surrounding front segment (13) as a result of withdrawal of said needle hub (25) from said surrounding front segment (13) ; and
a retaining member (128,235) disposed between said inner tubular wall surface (127) and said outer surrounding wall surface (233) to arrest unforced movement of said surrounding barrel wall (23) in the position of use, said retaining member includes male and female screw thread segments (128,235) which are disposed on said inner tubular wall surface (127) and said outer surrounding wall surface (233) respectively said male and female screw thread segments (128,235) being [and which are] angularly engageable once said encasing member (1) is brought over said needle hub (25) and is sleeved on said syringe body (2) so as to restrain said surrounding barrel wall (23) from displacing along said surrounding rear segment (12) in the longitudinal direction, thereby placing said surrounding barrel wall (23) in the position of use **characterised in that** said female screw thread segment (235) being a spirally extending groove

2. The disposable syringe of Claim 1, **characterized in that** said male and female screw thread segments (128,235) are configured such that said surrounding barrel wall (23) is angularly movable in a clockwise direction relative to said surrounding rear segment (12) so as to displace said surrounding barrel wall (23) from the position of use to the disposal position.

3. The disposable syringe of Claim 2, further **characterized by** a surrounding retaining groove (234) which is formed in said outer surrounding wall surface (233) adjacent to said front end wall (24) and which is opposite to said female screw thread segment (235) in the longitudinal direction so as to engage said male screw thread segment (128) when said surrounding barrel wall (23) is in the disposal position, thereby restraining said surrounding barrel wall (23) from displacing along said surrounding rear segment (12).

4. The disposable syringe of Claim 3, further **characterized by** a guideway (237) which is disposed on said outer surrounding wall surface (233), and which extends to interconnect said female screw thread segment (235) and said surrounding retaining groove (234) so as to facilitate the displacement of said surrounding barrel wall (23) from the position of use to the disposal position.

5. The disposable syringe of Claim 1, **characterized in that** said needle hub (25) is integrally formed with said front end wall (24) and extends from said front end wall (24) in the longitudinal direction.

6. The disposable syringe of Claim 5, **characterized in that** said surrounding front segment (13) has an inner front wall surface (131) with a diameter which is smaller than that of said inner tubular wall surface (127) so as to form a surrounding shoulder portion (14) therebetween, such that said front end wall (24) abuts against said surrounding shoulder portion (14) in the position of use.

7. The disposable syringe of Claim 6, **characterized in that** said needle hub (25) has a plurality of ribs (252) displaced angularly from one another about the axis (X), each of said ribs (252) extending radially and outwardly so as to create an increased friction force at said inner front wall surface (131), thereby establishing greater friction engagement between said surrounding front segment (13) and said needle hub (25) when said surrounding front segment (13) is sleeved on said needle hub (25).

8. The disposable syringe of Claim 1, **characterized in that** said surrounding rear segment (12) further has an outer tubular wall surface (125) radially opposite to said inner tubular wall surface (127), and a plurality of slots (15) disposed in said outer tubular wall surface (125) and extending through said inner tubular wall surface (127) so as to expose a portion of said surrounding barrel wall (23).

9. The disposable syringe of Claim 1, **characterized in that** said surrounding front segment (13) has an outer front wall surface which has a rib portion (133) extending in the longitudinal direction, said disposable syringe further comprising a tip protector (4) which has a sleeve end (41) disposed to sleeve on said outer front wall surface, said sleeve end (41) including a groove portion which mates with said rib portion (133) to result in a splined engagement between said tip protector (4) and said outer front wall surface, thereby ensuring secure shielding of said needle cannula (26).

## Patentansprüche

1. Einwegspritze umfassend:
einen Spritzenkörper (2) enthaltend
eine umgebende Zylinderwand (23), welche eine Achse (X), vordere und rückwärtige, in einer Längsrichtung parallel zu der Achse (X) einander gegenüberliegende, umgebende Enden (230, 232), eine äußere umgebende Wandoberfläche (233), und
eine innere umgebende Wandoberfläche (239) gegenüber der äußeren umgebende Wandoberfläche (233) aufweist, wobei die innere umgebende Wandoberfläche (239) eine Aufnahmekammer (21) begrenzt,
eine vordere Endwand (24), die sich von dem vorderen umgebende Ende (230) radial und in Richtung der Achse (X) erstreckt, um eine Innenöffnung (241) zu begrenzen, die einen Flüssigkeitsübertragungsweg mit der Aufnahmekammer (21) bildet,
eine Nadelkanüle (26), die angeordnet ist, um sich in Längsrichtung zu erstrecken, und eine Nadelnabe (25), die angeordnet ist, um die Nadelkanüle (26) an der vorderen Endwand (24) zu sichern, so dass die Nadelkanüle (26) mit dem Flüssigkeitsübertragungsweg mit der Aufnahmekammer (21) durch die Innenöffnung (241) in der Längsrichtung in Verbindung steht; und
einen Kolben (3), der einen Kopfabschnitt (31), der angeordnet ist, um in der Aufnahmekammer (21) bewegbar zu sein und in gleitbarem Kontakt mit der inneren umgebenden Wandoberfläche (239) steht, und einen Schaftabschnitt (32) enthält, der sich von dem Kopfabschnitt (31) in der Längsrichtung und nach außen von dem rückwärtigen umgebenden Ende (232) erstreckt, um manuell betätigbar zu sein, wobei die Einwegspritze **gekennzeichnet ist, durch**:
ein Umhüllungselement (1), welches so ausgestaltet ist, dass es als Hülle von der Nadelnabe (25) her auf den Spritzenkörper (2) aufzubringen ist, und welches ein umgebendes vorderes Segment (13) enthält, welches die Nadelnabe (25) umgibt, und welche es der Nadelkanüle (26) erlaubt, sich davon nach außen zu erstrecken und relativ dazu zurückziehbar zu sein, und
ein umgebendes rückwärtiges Segment (12), das sich von dem umgebenden vorderen Segment (13) in der Längsrichtung erstreckt, um auf die umgebenden Zylinderwand (23) als Hülle aufgebracht zu werden, und das eine innere rohrförmige Wandoberfläche (127) hat, wobei die innere rohrförmige Wandoberfläche (127) der äußeren umgebenden Wandoberfläche (233) gegenüberliegt und relativ dazu zwischen einer Verwendungsposition, wo die vordere Endwand (24) näher an dem umgebenden vorderen Segment (13) ist und wo die Nadelkanüle (26) sich von dem umgebenden vorderen Segment (13) nach außen erstreckt, und einer Entsorgungsposition bewegbar ist, wo die vordere Endwand (24) von dem umgebenden vorderen Segment (13) entfernt ist und wo die Nadelkanüle (26) innerhalb des umgebenden vorderen Segments (13) als ein Ergebnis des Rückzuges der Nadelnabe (25) von dem umgebenden vorderen Segment (13) angeordnet ist; und
ein Halteelement (128, 235), das zwischen der inneren rohrförmigen Wandoberfläche (127) und der äußeren umgebenden Wandoberfläche (233) angeordnet ist, um eine nicht erzwungene Bewegung der umgebenden Zylinderwand (23) in der Verwendungsposition aufzuhalten, wobei das Halteelement Außen- und Innengewinde-Segmente (128, 235) enthält, welche an der inneren rohrförmigen Wandoberfläche (127) beziehungsweise der äußeren umgebenden Wandoberfläche (233) angeordnet sind, wobei die Außen- und Innengewinde-Segmente (128, 235) winkelmäßig miteinander in Eingriff zu bringen sind [und es auch werden] sobald das Mantelelement (1) über die Nadelnabe (25) gebracht wurde und als Hülle auf den Spritzenkörper (2) aufgebracht wird, um die umgebende Zylinderwand (23) gegen eine Verschiebung entlang dem umgebenden rückwärtigen Segment (12) in der Längsrichtung zurückzuhalten, um **dadurch** die umgebende Zylinderwand (23) in der Verwendungsposition zu platzieren, **dadurch gekennzeichnet, dass** das Innengewinde-Segment (235) eine sich spiralförmig erstreckende Nut ist.

2. Einwegspritze nach Anspruch 1, **dadurch gekennzeichnet, dass** die Außen- und Innengewinde-Segmente (128, 235) derart ausgestaltet sind, dass die umgebende Zylinderwand (23) winkelmäßig in Uhrzeigerrichtung relativ zu dem umgebenden rückwärtigen Segment (12) bewegbar ist, um die umgebende Zylinderwand (23) von der Verwendungsposition zu der Entsorgungsposition zu bewegen.

3. Einwegspritze nach Anspruch 2, ferner **gekennzeichnet durch** eine umgebende Haltenut (234), die in der äußeren umgebenden Wandoberfläche (233) neben der vorderen Endwand (24) ausgebildet ist und welche in der Längsrichtung dem Innengewinde-Segment (235) gegenüberliegt, um das Außengewinde-Segment (128) zu erfassen, wenn sich die umgebende Zylinderwand (23) in der Entsorgungsposition befindet, um dabei die umgebende Zylinderwand (23) gegen eine Bewegung entlang dem umgebenden rückwärtigen Segment (12) zurückzuhalten.

4. Einwegspritze nach Anspruch 3, ferner **gekennzeichnet durch** einen Führungsweg (237), der an der äußeren umgebenden Wandoberfläche (233) angeordnet ist, und welcher sich erstreckt, um das Innengewinde-Segment (235) und die umgebende Haltenut (234) zu verbinden, um das Bewegen der umgebenden Zylinderwand (23) von der Verwendungsposition zu der Entsorgungsposition zu erleichtern.

5. Einwegspritze nach Anspruch 1, **dadurch gekennzeichnet, dass** die Nadelnabe (25) einteilig mit der vorderen Endwand (24) ausgebildet ist und sich von der vorderen Endwand (24) in der Längsrichtung erstreckt.

6. Einwegspritze nach Anspruch 5, **dadurch gekennzeichnet, dass** das umgebende vordere Segment (13) eine innere vordere Wandoberfläche (131) mit einem Durchmesser aufweist, der kleiner als jener der inneren rohrförmigen Wandoberfläche (127) ist, um einen umgebenden Schulterabschnitt (14) dazwischen auszubilden, so dass die vordere Endwand (24) an den umgebenden Schulterabschnitt (14) in der Verwendungsposition anstößt.

7. Einwegspritze nach Anspruch 6, **dadurch gekennzeichnet, dass** die Nadelnabe (25) eine Vielzahl von Rippen (252) aufweist, die winkelmäßig voneinander über die Achse (X) versetzt sind, wobei jede der Rippen (252) sich radial und nach außen erstreckt, um eine erhöhte Reibungskraft an der inneren vorderen Wandoberfläche (131) zu erzeugen, um **dadurch** einen größeren Reibungseingriff zwischen dem umgebenden vorderen Segment (13) und der Nadelnabe (25) aufzubauen, wenn das umgebende vordere Segment (13) auf die Nadelnabe (25) geschoben wird.

8. Einwegspritze nach Anspruch 1, **dadurch gekennzeichnet, dass** das umgebende rückwärtige Segment (12) ferner eine äußere rohrförmige, der inneren rohrförmigen Wandoberfläche (127) radial gegenüberliegende Wandoberfläche (125) und eine Vielzahl von Schlitzen (15) aufweist, die in der äußeren rohrförmigen Wandoberfläche (125) angeordnet sind und sich durch die innere rohrförmige Wandoberfläche (127) erstrecken, um einen Abschnitt der umgebenden Zylinderwand (23) freizulegen.

9. Einwegspritze nach Anspruch 1, **dadurch gekennzeichnet, dass** das umgebende vordere Segment (13) eine äußere vordere Wandoberfläche aufweist, welche einen Rippenabschnitt (133) aufweist, der sich in Längsrichtung erstreckt, wobei die Einwegspritze ferner einen Spitzenschutz (4) umfasst, der ein Hüllenende (41) aufweist, der angeordnet ist, um die äußere vordere Wandoberfläche zu umhüllen, wobei das Hüllenende (41) einen Nutabschnitt enthält, welcher in den Rippenabschnitt (133) einzufügen ist, so dass sich eine Keil-Befestigung zwischen dem Spitzenschutz (4) und der äußeren vorderen Wandoberfläche ergibt, um somit eine sichere Abschirmung der Nadelkanüle (26) sicherzustellen.

## Revendications

1. Seringue jetable, comprenant :
un corps (2) de seringue incluant :
une paroi (23) de corps d'entourage qui a un axe (X) , des extrémités avant et arrière (230, 232) d'entourage opposées l'une à l'autre dans une direction longitudinale parallèle à l'axe (X), une surface extérieure (233) de paroi d'entourage et une surface intérieure (239) de paroi d'entourage opposée à ladite surface extérieure (233) de paroi d'entourage, ladite surface intérieure (239) de paroi d'entourage confinant une chambre (21) de réception,
une paroi (24) d'extrémité avant qui s'étend radialement de ladite extrémité avant (230) d'entourage et en direction de l'axe (X) pour confiner un orifice intérieur (241) en communication fluidique avec ladite chambre (21) de réception,
une canule (26) d'aiguille disposée pour s'étendre dans la direction longitudinale, et
un moyeu (25) d'aiguille disposé pour fixer ladite canule (26) d'aiguille à ladite paroi (24) d'extrémité avant pour que, dans la direction longitudinale, ladite canule (26) d'aiguille soit en communication fluidique avec ladite chambre (21) de réception par l'intermédiaire dudit orifice intérieur (241) ; et
un plongeur (3) incluant une partie (31) de tête disposée pour pouvoir être mue dans ladite chambre (21) de réception et en contact coulissant avec ladite surface intérieure (239) de paroi d'entourage, et une partie (32) de tige s'étendant de ladite partie (31) de tête dans la direction longitudinale et vers l'extérieur de ladite extrémité arrière (232) d'entourage pour pouvoir être actionnée manuellement, ladite seringue jetable étant
**caractérisée par** :
un élément (1) de confinement qui est configuré pour pouvoir être manchonné sur ledit corps (2) de seringue à partir dudit moyeu (25) d'aiguille, et qui inclut un segment avant (13) d'entourage qui entoure ledit moyeu (25) d'aiguille, et qui permet à ladite canule (26) d'aiguille d'être déployée vers l'extérieur de celui-ci ou de pouvoir être rétractée par rapport à celui-ci, et
un segment arrière (12) d'entourage s'étendant dudit segment avant (13) d'entourage dans la direction longitudinale, prévu pour être manchonné sur ladite paroi (23) de corps d'entourage, et comportant une surface intérieure tubulaire (127) de paroi, ladite surface intérieure tubulaire (127) de paroi faisant face et étant mobile par rapport à ladite surface extérieure (233) d'entourage de paroi entre une position d'utilisation, dans laquelle ladite paroi (24) d'extrémité avant est plus proche dudit segment avant (13) d'entourage, et dans laquelle ladite canule (26) d'aiguille s'étend vers l'extérieur dudit segment avant (13) d'entourage, et une position de mise au rebut, dans laquelle ladite paroi (24) d'extrémité avant se trouve à distance dudit segment avant (13) d'entourage, et dans laquelle ladite canule (26) d'aiguille est disposée à l'intérieur dudit segment avant (13) d'entourage comme résultat d'un retrait dudit moyeu (25) d'aiguille dudit segment avant (13) d'entourage ; et
un élément (128, 235) de retenue disposé entre ladite surface intérieure tubulaire (127) de paroi et ladite surface extérieure (233) de paroi d'entourage à des fins d'arrêter un mouvement non forcé de ladite paroi (23) de corps d'entourage dans la position d'utilisation, ledit élément de retenue inclut des segments filetés mâle et femelle (128, 235) qui sont disposés respectivement sur ladite surface intérieure tubulaire (127) de paroi et sur ladite surface extérieure (233) de paroi d'entourage, lesdits segments filetés mâle et femelle (128, 235) pouvant engager angulairement [et étant engagés angulairement] lorsque ledit élément (1) de confinement est amené par dessus ledit moyeu (25) d'aiguille et est manchonné sur ledit corps (2) de seringue de façon à limiter un déplacement de ladite paroi (23) de corps d'entourage le long dudit segment arrière (12) d'entourage dans la direction longitudinale, en plaçant ainsi ladite paroi (23) de corps d'entourage dans la position d'utilisation, **caractérisée en ce que** ledit segment fileté femelle (235) est une rainure s'étendant en spirale.

2. Seringue jetable selon la revendication 1, **caractérisée en ce que** lesdits segments filetés mâle et femelle (128, 235) sont configurés de sorte que ladite paroi (23) de corps d'entourage peut bouger angulairement dans le sens des aiguilles d'une montre par rapport audit segment arrière (12) d'entourage de façon à pouvoir décaler ladite paroi (23) de corps d'entourage de la position d'utilisation à la position de mise au rebut.

3. Seringue jetable selon la revendication 2, **caractérisée en outre par** une rainure d'entourage (234) de retenue qui est formée dans ladite surface extérieure (233) de paroi d'entourage adjacente à ladite paroi (24) d'extrémité avant et qui est opposée audit segment fileté femelle (235) dans la direction longitudinale de façon à engager ledit segment fileté mâle (128) lorsque ladite paroi (23) de corps d'entourage est dans la position de mise au rebut, en limitant ainsi un déplacement de ladite paroi (23) de corps d'entourage le long dudit segment arrière (12) d'entourage.

4. Seringue jetable selon la revendication 3, **caractérisée en outre par** une glissière (237) qui est disposée sur ladite surface extérieure (233) de paroi d'entourage, et qui s'étend à des fins d'assurer une prise mutuelle entre ledit segment fileté femelle (235) et ladite rainure d'entourage (234) de retenue de façon à faciliter le déplacement de ladite paroi (23) de corps d'entourage de la position d'utilisation à la position de mise au rebut.

5. Seringue jetable selon la revendication 1, **caractérisée en ce que** ledit moyeu (25) d'aiguille est formé d'un seul tenant avec ladite paroi (24) d'extrémité avant et s'étend de ladite paroi (24) d'extrémité avant dans la direction longitudinale.

6. Seringue jetable selon la revendication 5, **caractérisée en ce que** ledit segment avant (13) d'entourage comporte une surface intérieure (131) de paroi avant qui a un diamètre plus petit que celui de ladite surface intérieure tubulaire (127) de paroi de façon à former, entre elles, une partie (14) d'épaulement d'entourage, de sorte que, dans la position d'utilisation, ladite paroi (24) d'extrémité avant vient en butée contre ladite partie (14) d'épaulement d'entourage.

7. Seringue jetable selon la revendication 6, **caractérisée en ce que** ledit moyeu (25) d'aiguille comporte une pluralité de nervures (252) décalées angulairement les unes des autres autour de l'axe (X), chacune desdites nervures (252) s'étendant radialement et vers l'extérieur de façon à créer une force de frottement accrue au niveau de ladite surface intérieure (131) de paroi avant, en établissant ainsi un engagement de frottement plus puissant entre ledit segment avant (13) d'entourage et ledit moyeu (25) d'aiguille lorsque ledit segment avant (13) d'entourage est manchonné sur ledit moyeu (25) d'aiguille.

8. Seringue jetable selon la revendication 1, **caractérisée en ce que** ledit segment arrière (12) d'entourage comporte en outre une surface extérieure tubulaire (125) de paroi opposée radialement à ladite surface intérieure tubulaire (127) de paroi, et une pluralité de fentes (15) disposées dans ladite surface extérieure tubulaire (125) de paroi et s'étendant à travers ladite surface intérieure tubulaire (127) de paroi de façon à exposer une partie de ladite paroi (23) de corps d'entourage.

9. Seringue jetable selon la revendication 1, **caractérisée en ce que** ledit segment avant (13) d'entourage comporte une surface extérieure de paroi avant qui comporte une partie nervurée (133) s'étendant dans la direction longitudinale, ladite seringue jetable comprenant en outre un dispositif de protection (4) de pointe qui comporte une extrémité (41) de manchon disposée pour être manchonnée sur ladite surface extérieure de paroi avant, ladite extrémité (41) de manchon incluant une partie rainurée qui s'accouple avec ladite partie nervurée (133) pour établir un engagement par cannelures entre ledit dispositif de protection (4) de pointe et ladite surface extérieure de paroi avant, en garantissant ainsi une protection sûre de ladite canule (26) d'aiguille.
